Europäisches Patentamt

European Patent Office

Office européen des brevets

(11) Publication number: **0 487 028 A2**

## EUROPEAN PATENT APPLICATION

(21) Application number: **91119723.4**

(22) Date of filing: **19.11.91**

(51) Int. Cl.5: **G01N 1/28**, B01L 3/00, B01D 63/08, B01D 61/18, B01D 29/01

(30) Priority: **22.11.90 JP 320223/90**

(43) Date of publication of application:
**27.05.92 Bulletin 92/22**

(84) Designated Contracting States:
**DE GB**

(71) Applicant: **SHIMADZU CORPORATION**
**1, Nishinokyo-Kuwabaracho**
**Nakagyo-ku Kyoto-shi Kyoto 604(JP)**

(72) Inventor: **Yamagata, Ko-ichi**
**11-25, 1-chome, Komatsu,**
**Higashiyodogawa-ku**
**Osaka-shi, Osaka-fu(JP)**
Inventor: **Shirasaki, Yoshinari**
**3-25-13, Shigasato**
**Otsu-shi, Shiga-ken(JP)**
Inventor: **O-Hashi, Tetsuo**
**18, Shunei-cho, Saiin, Ukyo-ku**
**Kyoto-shi, Kyoto-fu(JP)**
Inventor: **Tada, Jun**
**16, Nanjyo, Mozume-cho**
**Mukou-shi, Kyoto-fu(JP)**
Inventor: **Fukushima, Shigeru**
**4-25-5, Ichiriyama Otsu-shi**
**Shiga-ken(JP)**

(74) Representative: **Vossius & Partner**
**Siebertstrasse 4 P.O. Box 86 07 67**
**W-8000 München 86(DE)**

(54) **Apparatus for extracting and purifying nucleic acid.**

(57) The present invention is directed to an apparatus for extracting and purifying nucleic acid comprising: a group of vessels containing at least a solution for nucleic acid extraction and purification; means (1,2) for aspirating and discharging said solution arranged so that it is movable among said vessels; and means for attaching a filter unit (41) to a solution aspiration/discharge portion of said means (1,2) for aspirating and discharging the solution.

The apparatus of the present invention makes it possible to automatically perform extraction and purification of the nucleic acid component and is very useful in quick, simple and safe extraction and purification thereof in such a field as clinical examination, where a large number of samples are dealt with.

FIG.1

The present invention relates to an apparatus for extracting and purifying nucleic acid, more specifically to an apparatus for extracting and purifying the nucleic acid component from a microbe or cell contained in the biological sample.

In the fields of clinical examination and research, it has been a common practice to separate the nucleic acid component from a microbe or cell and determine its base sequence by hybridization assay or PCR [polymerase chain reaction; Saiki, R. K. et al., Science 239, 487-491 (1988)] for the purpose of identifying the microbial species in biological sample or clarifying the nature of the microbe or cell.

In the conventional method of obtaining the nucleic acid component from a microbe in a sample containing water-soluble solids (e.g., food residues, peeled-off cells in the intestine), such as feces, urine, blood or cerebrospinal fluid as the biological sample, a sample suspension is cultivated in medium for several days, and the resulting colonies are collected and centrifuged to separate and recover bacterial cells. These cells are then lysed with an enzyme such as proteinase K, a surfactant such as sodium dodecyl sulfate or an alkali such as sodium hydroxide, or disrupted by a physical disruption means such as an ultrasonic wave or mill to yield the microbe-derived nucleic acid component.

The nucleic acid component thus obtained has been purified by phenol/chloroform treatment and ethanol precipitation, which require centrifugation and troublesome manual operation ["DNA Probe II", Toyozo Takahashi, CMC Shuppan (1990)].

In the fields of clinical examination and research, obtaining genetic information from the nucleic acid component of a microbe or cell leads to clarification of the essential nature of the microbe or cell and is hence useful for exact identification of the microbe or cell and diagnosis of the infectious disease. However, this method of identification and diagnosis based on the nucleic acid component has not yet been widely used despite its utility, since there is no apparatus for simply and quickly extracting and purifying the nucleic acid component from a microbe or cell in the sample.

Another problem in the conventional method is that workers who deal with samples containing a pathogenic microbe are constantly subject to a risk of bio-hazard such as infectious disease because all procedures are based on manual operation.

It is an object of the present invention to provide an apparatus for simply, quickly and safely extracting and purifying the microbe- or cell-derived nucleic acid from a biological sample such as feces, urine, blood or cerebrospinal fluid.

Aiming at solving the problems described above, the present invention is characterized by the provision of at least a group of vessels containing a solution for nucleic acid extraction and purification, means for aspirating and discharging the solution arranged so that it is movable among the vessels and means for attaching a filter unit to the solution aspiration/discharge portion of the means for aspirating and discharging the solution.

BRIEF DESCRIPTION OF THE DRAWINGS

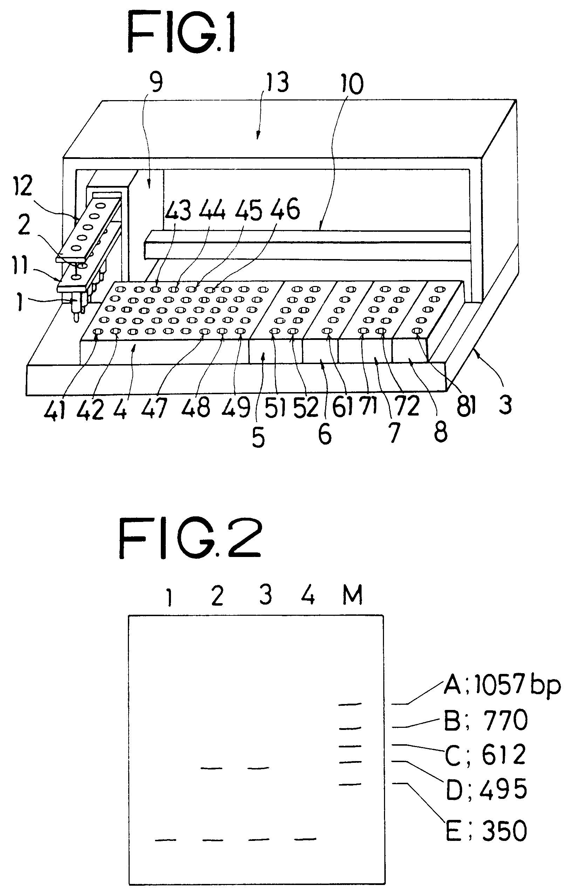

Figure 1 is a schematic diagram of a mode of embodiment of the apparatus for extracting and purifying nucleic acid of the present invention. Figure 2 is electrophoretic patterns. The numerical symbols in these figures denote a syringe (1), a piston (2), a table plate (3) and a tray (4), respectively.

DETAILED DESCRIPTION OF THE INVENTION

In the present invention, the solution for nucleic acid extraction and purification may be each of a detergent, buffer, enzyme solution, lysate, extract, etc., and its kind is determined depending on the sample subjected to extraction and purification.

Although the group of vessels must contain at least the solution of nucleic acid extraction and purification described above, it may also contain a sample solution and may also be kept empty for discharging the solution.

Furthermore, the group of vessels may be provided with means for heating and cooling.

The group of vessels is exemplified by a number of sample tubes arranged in a tray or aluminum block, but this is not to be construed as limitative.

When using an aluminum block, it can easily be heated or cooled by using a heating unit or Peltier element embedded therein or by flowing a fluid such as water, polyethylene glycol, silicon oil, ice or freon.

The means for aspirating and discharging the solution is exemplified by an assembly of a syringe and a piston housed therein which aspirates and discharges the solution by the action of the piston, but this is not to be construed as limitative. Although it is preferable to use a motor to drive the piston, this is not to be construed as limitative and the piston may be driven by oil pressure or gas pressure.

Transfer of the means for aspirating and discharging the solution among the vessels is preferably achieved by a motor, but this is not to be construed as limitative and the transfer may be achieved by oil pressure or gas pressure.

When a syringe, for example, is used as the means for aspirating and discharging the solution, the means for attaching a filter unit to the portion of aspiration/discharge is exemplified by the method in which the syringe and the filter unit are each provided with a taper and the syringe is pushed into the filter unit, but this is not to be construed as limitative; it may be the method in which the syringe opening and the filter unit junction are screwed together or the method in which the syringe and the filter unit are locked by a claw provided on either of them.

Although it is preferable from the viewpoint of continuous operation of sampling, extraction and purification to house the filter unit for the present invention in the group of vessels described above, the filter unit may be outside the group of vessels.

The filter in the filter unit for the present invention is preferably a glass filter having a pore size of about 0.5 to 1.0 $\mu$m, but this is not to be construed as limitative.

In the apparatus of the present invention, it is preferable that action of the means for aspirating and discharging the solution, attaching the filter unit and other operations all be controlled as a series of actions by a computer in the control console using a program which can easily be partially modified, but this is not to be construed as limitative.

The apparatus for extracting and purifying nucleic acid of the present invention makes it possible to automatically perform extraction and purification of the nucleic acid component in as short as about 1.5 hours, which require much labor, trouble and time (over half day) if performed by the conventional method. In addition, the use of this apparatus obviates direct contact with the sample during operation, thus ensuring safe obtainment of the nucleic acid component even from samples containing a pathogenic microbe or the like.

Therefore, the apparatus for extracting and purifying nucleic acid of the present invention is very useful in quick, simple and safe extraction and purification of the nucleic acid component in the fields of clinical examination, food examination and research, where a large number of samples are dealt with.

EXAMPLE

The present invention is hereinafter described in more detail by means of the following example, but the invention is not limited by the example.

Figure 1 shows a schematic diagram of a mode of embodiment of the apparatus for extracting and purifying nucleic acid of the present invention. A syringe 1 is fixed on a syringe table 11, and a piston 2 is fixed on a piston table 12. The syringe table 11 and the piston table 12 can be independently moved up and down using a motor. The syringe table 11 and the piston table 12 together with a supporting table 9 can be separately moved to right and left along a guide 10.

On a base board 3, a tray 4, an aluminum block 5, an aluminum block 6, an aluminum block 7 and an aluminum block 8 are fixed, of which the aluminum blocks 6, 7 and 8 are controlled to be kept at 37 °C, 60°C and 95°C, respectively, by heating by a heating unit. The aluminum block 5 is controlled to be kept at 0°C by cooling by a Peltier element.

The tray 4 contains a number of filter unit 41, sample tube 42, detergent-containing sample tube 43, detergent-containing sample tube 44, buffer-containing sample tube 45, sample tube 46 containing a lytic solution A, sample tube 47 containing a lytic solution B, sample tube 48 containing 99% ethanol and eluent-containing sample tube 49, and the number of each of them is the same as the number of syringe 1.

The aluminum block 5 contains a number of sample tube 51 containing 95% ethanol (pH 5.2) containing 0.15 M sodium acetate and sample tube 52 containing 70% ethanol, and the number of each of them is the same as the number of syringe 1.

The aluminum blocks 6 and 8 each contain a number of empty sample tube 61 and empty sample tube 81, respectively, and the number of each of them is the same as the number of syringe 1.

The aluminum block 7 contains a number of empty sample tube 71 and empty sample tube 72, and the number of each of them is the same as the number of syringe 1.

Extraction and purification of the nucleic acid component from fecal samples

To 1 g of feces from a normal human, 9 ml of a 50 mM phosphate buffer was added and the feces was suspended. Further to this suspension, $10^6$ cells of S. aureus cultured separately were added and suspended well to yield a sample.

This fecal sample suspension was filtered through a nylon mesh filter of 10 $\mu$m in pore size and a glass filter of 2.7 $\mu$m in pore size to remove coarse impurities such as food residues. Then, using the apparatus for extracting and purifying nucleic acid illustrated in Figure 1, which is a mode of embodiment of the present invention, the nucleic acid component of S. aureus in the fecal sample suspension was extracted and purified in accordance with the procedures described in the following steps 1 through 17.

Step 1: Sample aspiration into syringe

0.5 ml of the fecal sample suspension treated with the nylon mesh filter to remove coarse impurities as above was transferred to the sample tube 42.

After fixing the syringe 1 onto the syringe table 11 and fixing the piston 2 onto the piston table 12, the syringe 1 was transferred above the sample tube 42. Subsequently, the syringe table 11 and the piston table 12 were lowered until the opening of the syringe became in contact with the base of the sample tube 42. Then, the piston table 12 was pulled up to aspirate the fecal sample solution in the sample tube 42 into the syringe. Finally, the syringe table 11 and the piston table 12 were pulled up to raise the syringe 1.

Step 2: Attaching the filter unit

Through the filter unit 41 incorporating a layered combination of a glass filter of 4 mm in diameter and 0.6 $\mu$m in pore size and another glass filter of 1 $\mu$m in pore size, a detergent (a 50 mM phosphate buffer, pH 7.0, containing surfactants Nonidet P-40 and Tween 20 at 0.05% for each) was passed to moisten the glass filters.

The syringe 1 was transferred above the filter unit 41, and the syringe table 11 and the piston table 12 were pressed down to attach the filter unit 41 to the opening of the syringe 1. Finally, the syringe 1 together with the filter unit 41 was pulled up.

Step 3: Trapping of bacterial cells

After transferring the syringe 1 above the sample tube 42 and lowering the syringe 1 until the tip of the filter unit 41 came near the opening of the sample tube 42, the piston 2 was pressed down to discharge the fecal sample solution into the sample tube 42, upon which cells of S. aureus in the fecal sample solution were trapped by the filters in the filter unit 41.

Step 4: Washing of bacterial cells

After transferring the syringe 1 above the sample tube 43 containing 1 ml of the detergent and lowering the syringe 1 until the filter unit reached the base of the sample tube 43, the piston 2 was pulled up to aspirate the detergent. Then, after pulling up the syringe 1 until the tip of the filter unit 41 came near the opening of the sample tube 43, the piston 2 was pressed down to discharge the detergent into the sample tube 43, upon which cells of S. aureus were washed by the detergent.

Step 5: Washing of bacterial cells

Using the sample tube 44, the same procedure as in step 4 was carried out.

Step 6: Removal of detergent

After transferring the syringe 1 above the sample tube 45 containing 1 ml of a 50 mM phosphate buffer and lowering the syringe 1 until the filter unit 41 reached the base of the sample tube 45, the piston 2 was pulled up to aspirate the buffer. Then, after pulling up the syringe 1 until the tip of the filter unit 41 came near the opening of the sample tube 43, the piston 2 was pressed down to discharge the buffer into the sample tube 45, upon which the solutions around the filters in the filter unit 41 and the detergent around the cells were replaced with the 50 mM phosphate buffer.

Step 7: Aspiration of the lytic solution A

After transferring the syringe 1 above the sample tube 46 containing 100 $\mu$l of the lytic solution A (a 50 mM phosphate buffer, pH 7.0, containing 1 mg/ml achromopeptidase and 50 $\mu$g/ml N-acetyl-muramidase) and lowering the syringe 1 until the tip of the filter unit 41 reached the base of the sample tube 46, the piston 2 was pulled up to aspirate the lytic solution A. In this procedure, the distance of raising the piston 2 was adjusted so that the vicinities of the glass filters in the filter unit 41 were filled with the lytic solution A.

Step 8: Bacteriolysis

While retaining the lytic solution A in the syringe 1, the syringe 1 was transferred above the sample tube 61 on the aluminum block 6 being kept at 37°C, and the syringe 1 was lowered until the tip of the filter unit 41 reached the base of sample tube 61 while retaining the lytic solution A, followed by incubation under constant conditions for 10 minutes. After incubation, the piston 2 was pulled up to aspirate the lytic solution A from the syringe 1 into the syringe 1.

Step 9: Aspiration of the lytic solution B

While retaining the lytic solution A in the syringe 1, the syringe 1 was transferred above the sample tube 47 containing 100 $\mu$l of the lytic solution B (a 50 mM phosphate buffer, pH 7.0, containing 2 mg/ml proteinase K and 2% SDS), and the syringe 1 was lowered until the tip of the filter unit 41 reached the base of the sample tube 47, the piston 2 was pulled up to aspirate the lytic solution B. In this procedure, the distance of raising the piston 2 was adjusted so that the vicinities of the glass filters in the filter unit 41 were filled with the lytic solution B.

Step 10: Bacteriolysis

While retaining the lytic solution mixture in the syringe 1, the syringe 1 was transferred above the sample tube 71 on the aluminum block 7 being kept at 60°C, and the syringe 1 was lowered until the tip of the filter unit 41 reached the base of the sample tube 71 while retaining the lytic solution mixture, followed by incubation under constant conditions for 30 minutes. After incubation, the piston 2 was pulled up to aspirate the lytic solution mixture from the syringe 1 into the syringe 1.

Step 11: Enzyme inactivation

While retaining the lytic solution mixture in the syringe 1, the syringe 1 was transferred above the sample tube 81 on the aluminum block 8 being kept at 95°C, and the syringe 1 was lowered until the tip of the filter unit 41 reached the base of the sample tube 81 while retaining the lytic solution mixture, followed by incubation under constant conditions for 10 minutes. After incubation, the piston 2 was pulled up to aspirate the lytic solution mixture from the syringe 1 into the syringe 1.

Step 12: Precipitation of nucleic acid component with ethanol

While retaining the lytic solution mixture in the syringe 1, the syringe 1 was transferred above the sample tube 51 (containing 420 $\mu$l of a 95% ethanol solution, pH 5.2, containing 0.15 M sodium acetate) on the aluminum block 5 being kept at 0°C, and the syringe 1 was lowered until the tip of the filter unit 41 reached the base of the sample tube 51. Then, the piston 2 was raised to aspirate the ethanol solution of sodium acetate in the sample tube into the syringe 1. Subsequently, the piston 2 was pressed down to discharge the solution in the syringe 1 into the sample tube 51, upon which the nucleic acid component became insoluble in the syringe and was trapped by the filters.

Step 13: Washing of nucleic acid component

The syringe 1 was transferred above the sample tube 52 (containing 1 ml of 70% ethanol) on the aluminum block 5 being kept at 0°C, and the syringe 1 was lowered until the tip of the filter unit 41 reached the base of the sample tube 52. Then, the piston 2 was raised to aspirate the 70% ethanol in the sample tube 52 into the syringe 1. Subsequently, the piston 2 was pressed down to discharge the solution in the syringe 1 into the sample tube 52, upon which the nucleic acid component which had been trapped on the filters in an insoluble form was washed by ethanol.

Step 14: Washing of nucleic acid component

The syringe 1 was transferred above the sample tube 48 (containing 1 ml of 99% ethanol) on the tray 4, and the syringe 1 was lowered until the tip of the filter unit 41 reached the base of the sample tube 48. Then, the piston 2 was raised to aspirate the 99% ethanol in the sample tube 48 into the syringe 1. Subsequently, the piston 2 was pressed down to discharge the solution in the syringe 1 into the sample tube 48, upon which the nucleic acid component which had been trapped on the filters in an insoluble form was washed by ethanol as the same procedure as Step 13.

Step 15: Aspiration of eluent

The syringe 1 was transferred above the sample tube 49 containing 100 $\mu$l of an eluent (an aqueous solution containing 0.05% Nonidet P-40 and 0.05% Tween 20) on the tray 4, and the syringe 1 was lowered until the tip of the filter unit 41 reached the base of the sample tube 49. Then, the piston 2 was raised to aspirate the eluent in the sample tube 49 into the syringe 1. In this procedure, the distance of pulling up the piston 2 was adjusted so that the aspirated eluent may soak through the glass filters in the filter unit 41.

Step 16: Elution of nucleic acid component

While retaining the eluent in the syringe 1, the syringe 1 was transferred above the sample tube 72 on the aluminum block 7 being kept at 60°C, and the syringe 1 was lowered until the tip of the filter unit 41 reached the base of the sample tube 72 while retaining the eluent, followed by incubation under constant conditions for 10 minutes. After incubation, the piston 2 was pulled up to aspirate the eluent from the syringe 1 into the syringe 1, upon which the nucleic acid component which had been in an insoluble form was eluted in the eluent.

Step 17: Discharge of the nucleic acid solution

While retaining the nucleic acid solution in the syringe 1, the syringe 1 was transferred above the sample tube 49 which had contained the eluent on the tray 4, and the syringe 1 was lowered until the tip of the filter unit 41 reached the base of the sample tube 49 while retaining the nucleic acid solution. Subsequently, the piston 2 was pressed down to discharge the nucleic acid solution in the syringe 1 into the sample tube 49.

Detection of DNA derived from S. aureus

An aliquot of the nucleic acid solution obtained in the step 17 above was subjected to PCR under the following conditions. A 30 $\mu$l mixture of 3 $\mu$l of

the sample nucleic acid solution, 13.2 $\mu$l of distilled water, 4.8 $\mu$l of dNTP (each 1.25 mM dNTP), 1.5 $\mu$l of each of two kinds of primer (final concentration 20 $\mu$M), 3 $\mu$l of a 10-fold diluted buffer (produce by Perkin-Elmer Cetus) and 1.5 U of Taq polymerase (Perkin-Elmer Cetus) was layered with 100 $\mu$l of mineral oil as an anti-evaporation agent and set on a DNA Thermal Cycler (Perkin-Elmer Cetus). The 10-fold diluted buffer is a 100 mM Tris-HCl buffer (pH 9.0) containing 500 mM KCl and 15 mM $MgCl_2$. The primers used were synthetic oligonucleotides having base sequences specific to S. aureus described by Ohashi et al. in PTN01189547 using an automated DNA synthesizer (Cyclon Plus DNA Synthesizer, produced by MilliGen/Biosearch) and purifying them using a high performance liquid chromatograph equipped with a reverse phase column. A total of 42 PCR cycles were run, with denaturation at 94°C for 1 min, primer annealing at 50°C for 1 min, and elongation at 72°C for 1 min.

After completion of the PCR reaction described above, 8 $\mu$l of the reaction liquid was subjected to 35 minutes of electrophoresis at 100 V on a 2% agarose gel containing 0.5 $\mu$g/ml ethidium bromide, after which the gel was placed on a transilluminator and irradiated with a light of 320 nm wavelength. The DNA amplified by PCR reaction emitted fluorescence, which was photographed using a camera loaded with an instant film. The patterns thus obtained are shown in Figure 2. In Figure 2, lane 1 shows the pattern obtained by PCR of the nucleic acid solution obtained by treating a fecal sample free of S. aureus using the apparatus for extracting and purifying nucleic acid of the present invention; lane 2 shows the pattern obtained by PCR of the nucleic acid solution obtained by treating a fecal sample containing $10^6$ cells of S. aureus per gram of feces using the apparatus for extracting and purifying nucleic acid of the present invention; lane 3 shows the pattern obtained from a positive control for S. aureus; lane 4 shows the pattern obtained from a negative control; lane M shows the pattern obtained from the molecular weight marker obtained by completely digesting the $\phi$ x174 DNA with the restriction enzyme Hinc II; A, B, C, D and E are the numbers of corresponding base pairs. As a result, a band appeared on lane 2 at the position corresponding to about 486 base pairs. Because this position is the same as with the positive control sample (lane 3), which was electrophoresed at the same time, it is evident that S. aureus could be specifically detected. The positive control used was the nucleic acid solution obtained by manually lysing a pure culture of S. aureus in liquid medium with an enzyme and surfactant and then subjecting it to phenol and chloroform extraction and ethanol precipitation.

These results demonstrate that 335 base pairs of DNA was amplified without inhibiting the PCR reaction even with respect to the nucleic acid solution extracted and purified using a mode of embodiment of the apparatus of the present invention.

Also, when the nucleic acid component was extracted and purified from samples containing varied concentrations of S. aureus using the apparatus described above and detection of the DNA derived from S. aureus was attempted by hybridization assay, detection was possible though the sensitivity was lower than that obtained in detection by the PCR method.

From these findings, it is evident that the nucleic acid component derived from S. aureus in fecal samples was successfully extracted and purified.

**Claims**

1. An apparatus for extracting and purifying nucleic acid comprising: a group of vessels containing a solution for nucleic acid extraction and purification; means for aspirating and discharging said solution arranged so that it is movable among said vessels; and means for attaching a filter unit to a solution aspiration/discharge portion of said means for aspirating and discharging the solution.

2. The apparatus according to claim 1 wherein said group of vessels contain at least each of a detergent, buffer, enzyme solution, lysate, and extract.

3. The apparatus according to claim 1 or 2 wherein said group of vessels are provided with means for heating and cooling.

4. The apparatus according to any of claims 1 to 3 wherein said means for aspirating and discharging the solution is a syringe and a piston housed therein which aspirates and discharges the solution by the action of the piston.

5. The apparatus according to any of claims 1 to 4 wherein said means for attaching a filter unit to the solution aspiration/discharge portion of the means for aspirating and discharging the solution is a method in which the syringe and the filter unit are each provided with a taper and the syringe is pushed into the filter unit.

6. A process for extracting and purifying nucleic acid using the apparatus of any one of claims 1 to 5, comprising the steps of: aspirating a sample containing nucleic acid into a syringe; attaching a filter unit to the syringe; trapping bacterial cells in the filter unit; washing said bacterial cells by a detergent; removing said detergent; aspirating a lytic solution into said bacterial cells and bacteriolysis; inactivating an enzyme in said lytic solution; precipitating a nucleic acid component in said solution by ethanol; washing said nucleic acid component; aspirating an eluent and eluting said nucleic acid component; and discharging a nucleic acid solution.

# FIG.1

# FIG.2

A; 1057 bp
B; 770
C; 612
D; 495
E; 350